# EUROPEAN PATENT APPLICATION

(11) **EP 3 747 484 A1**
(43) Date of publication of application: **09.12.2020**
(21) Application number: 18903404.4
(22) Date of filing: 10.12.2018
(51) Int. Cl.: A61M 5/158

(54) **NEEDLE DEVICE**

(30) Priority: 01.02.2018 JP 2018016647
(71) Applicant: JMS Co., Ltd., Hiroshima 730-8652 (JP)
(72) Inventor: LIM Heng Jew, deceased (SG); MANI Prakash, Singapore 569620 (SG); CHONG Swee Shiong, Singapore 569620 (SG); TAY Yi Ling, Singapore 569620 (SG)
(74) Representative: Dehns
(86) International application number: PCT/JP2018/045235
(87) International publication number: WO 2019/150768

(57) **Abstract**

A needle device (1) is configured such that a needle unit (10) can be retracted into and contained in a protector (50) from a hub (15) side of the needle unit (10). In a state in which a cover (30) is not attached to the needle unit (10), once the needle unit (10) is contained in the protector (50), a locking claw (55) provided on the protector (50) engages with the hub (15) so that the needle unit (10) cannot be separated from the protector (50). In a state in which the cover (30) is attached to the needle unit (10), the needle unit (10) can be inserted into and removed from the protector (50).

## Description

### Technical Field

The present invention relates to a needle device including an injection needle with a sharp tip.

### Background Art

Injection needles are inserted into patients to perform blood collection, blood transfusion, infusion, or the like. There is a need to prevent accidental puncture of a medical personnel's finger or the like by a used injection needle after withdrawal from a patient (accidental needle stick).

Patent Document 1 discloses a needle device including a needle unit (referred to as a "hollow needle assembly" in Patent Document 1) in which a base end of an injection needle (referred to as a "hollow needle" in Patent Document 1) is supported by a hub, and a cylindrical protector (referred to as a "hollow needle assembly containing member" in Patent Document 1). A tube that is in communication with the injection needle is connected to a base end of the hub. The tube penetrates the protector. After the injection needle is withdrawn from a patient, the tube is pulled to retract the needle unit into the protector. An engagement portion provided in the protector engages with the hub. A cover (referred to as a "protector" in Patent Document 1) is put on the injection needle. The used injection needle is disposed of in a state of being contained in the protector and covered with the cover.

### Prior Art Documents

### Patent Document

[Patent Document 1] JP 2000-342686A

### Disclosure of Invention

### Problem to be Solved by the Invention

In the above-described needle device of Patent Document 1, when the needle unit is contained in the protector, the engagement portion of the protector engages with the hub of the needle unit. It is stated that the engaging force of the engagement portion of the protector with the hub is such that it restricts removal of the needle unit from the protector even when the tube is strongly pushed toward the needle unit in the state in which the needle unit is contained in the protector.

Accordingly, if the needle unit of an unused needle device is accidentally contained in the protector, the needle unit cannot be pulled out of the protector, and it is no longer possible to use the needle device.

An object of the present invention is to prevent accidental needle sticks of used injection needles, as well as operating mistakes that make unused needle devices no longer usable.

### Means for Solving Problem

A needle device of the present invention includes a needle unit having an injection needle with a sharp tip, and a hub holding a base end of the injection needle; a cover detachably attached to the needle unit so as to contain the injection needle; and a hollow, cylindrical protector having a locking claw. The needle device is configured such that the needle unit can be retracted into and contained in the protector from the hub side. In a state in which the cover is not attached to the needle unit, once the needle unit is contained in the protector, the locking claw engages with the hub so that the needle unit cannot be separated from the protector. In a state in which the cover is attached to the needle unit, the needle unit can be inserted into and removed from the protector.

### Effects of the Invention

The needle device of the present invention is configured such that, in the state in which the cover is not attached to the needle unit, once the needle unit is contained in the protector, the needle unit cannot be separated from the protector. Therefore, after use of the needle device, accidental needle stick with the used injection needle can be prevented by retracting the needle unit into the protector without reattaching the cover to the needle unit.

On the other hand, the needle device is configured such that, in the state in which the cover is attached to the needle unit, the needle unit can be inserted into and removed from the protector. Therefore, even if the needle unit before use to which the cover is attached is contained in the protector, a situation in which the needle device becomes unusable does not arise.

### Brief Description of Drawings

[FIG. 1] FIG. 1 is an exploded perspective view of a needle device according to an embodiment of the present invention.
[FIG. 2] FIG. 2A is a perspective view of a needle unit constituting the needle device according to the embodiment of the present invention, and FIG. 2B is a cross-sectional view of a hub taken along a plane containing line 2B-2B in FIG. 2A
[FIG. 3] FIG. 3A is a side view of the needle unit, and FIG. 3B is a top view of the needle unit.
[FIG. 4] FIG. 4 is a cross-sectional perspective view of a protector constituting the needle device according to the embodiment of the present invention.
[FIG. 5] FIG. 5 is a perspective view showing the needle device according to the embodiment of the present invention before the needle device is used.
[FIG. 6] FIG. 6 is a perspective view showing the needle device according to the embodiment of the present invention in a state in which a cover has been removed.
[FIG. 7] FIG. 7 is a cross-sectional view showing a state in which a used needle unit is in the process of being retracted into a protector.
[FIG. 8] FIG. 8 is a cross-sectional view showing a state in which the used needle unit is contained in the protector.
[FIG. 9] FIG. 9 is a cross-sectional view showing a state in which an unused needle unit is contained in the protector.
[FIG. 10] FIG. 10 is a perspective view showing the state in which the unused needle unit is contained in the protector.

### Description of the Invention

In the above-described needle device of the present invention, the locking claw may have a cantilevered structure including a free end that is a leading end of the locking claw and a fixed end that is arranged forward of the free end. The locking claw may slope from the fixed end toward the free end in an inward radius direction. With this configuration, the locking claw that engages with the hub when the needle unit to which the cover is not attached is contained in the protector can be realized with a simple configuration.

The locking claw may be elastically deformable such that the leading end thereof is displaced outward in a radius direction. With this configuration, the locking claw that engages with the hub can be realized with a simple configuration.

The hub may have a shoulder portion that adjoins a rear end of the cover when the cover is attached to the needle unit. The shoulder portion may be configured such that, in a state in which the cover is not attached to the needle unit, the locking claw engages with the shoulder portion. In a state in which the cover is attached to the needle unit, the rear end of the cover may protrude further outward in a radius direction than the shoulder portion. With this configuration, the needle device of the present invention in which whether or not the needle unit can be removed from the protector depends on whether or not the cover is attached to the needle unit can be realized with a simple configuration.

An outer circumferential surface of the hub may be provided with a rib extending in a direction in which the needle unit moves relative to the protector. The rib may be configured such that, when the needle unit moves within the protector, the locking claw slides on the rib. With this configuration, the movement of the needle unit within the protector is facilitated with a simple configuration in which the outer circumferential surface of the hub is provided with the rib.

The outer circumferential surface of the hub provided with the rib may be provided with a protruding and recessed shape for preventing slipping when gripped. In this case, the rib may protrude further outward in a radius direction than the protruding and recessed shape. With this configuration, both the grippability of the hub and the ease of movement of the needle unit within the protector can be achieved.

A sloping surface may be provided near a rear end of an outer circumferential surface of the hub, the sloping surface sloping forward in a direction away from a central axis. The sloping surface may be configured such that, when the needle unit moves within the protector, the locking claw slides on the sloping surface. With this configuration, the force that is needed when retracting the needle unit into the protector can be reduced.

The needle device of the present invention may further include a flexible tube connected to a rear end of the hub so as to be in communication with the injection needle. The tube may penetrate the protector. With this configuration, the needle unit can be inserted into and removed from the protector using the tube that is in communication with the injection needle. The insertion and removal of the needle unit can be performed without touching the injection needle, and safety is therefore improved.

Hereinafter, the present invention will be described in detail while presenting a preferred embodiment thereof. However, it goes without saying that the present invention is not limited to the embodiment below. The drawings that will be referred to in the following description show main members constituting the embodiment of the present invention in a simplified manner for the sake of convenience of description. Accordingly, the present invention may include optional members that are not shown in the drawings below. Also, the members shown in the drawings below may be changed or omitted within the scope of the present invention.

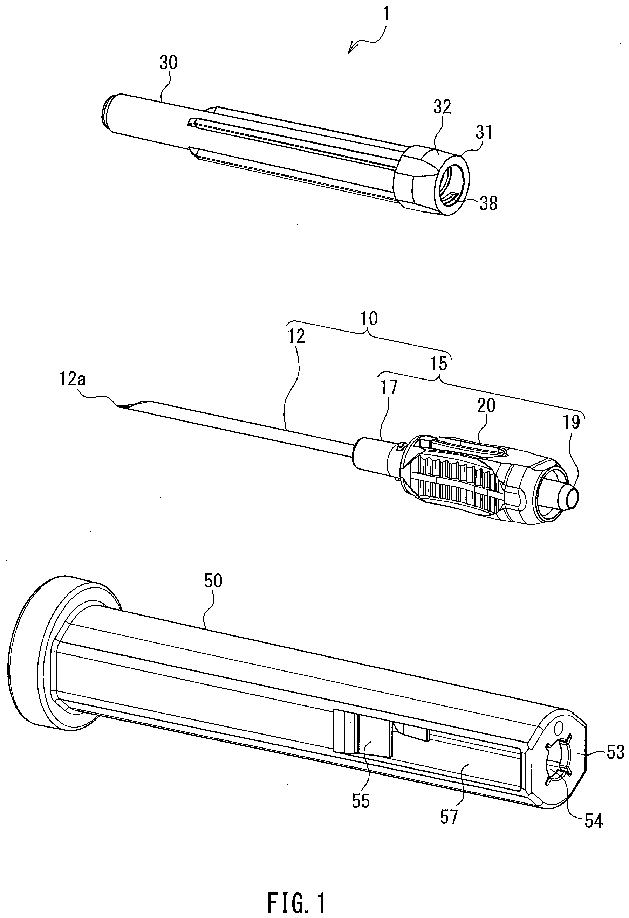

FIG. 1 is an exploded perspective view of a needle device 1 according to an embodiment of the present invention. The needle device 1 includes a needle unit 10, a cover 30, and a protector 50.

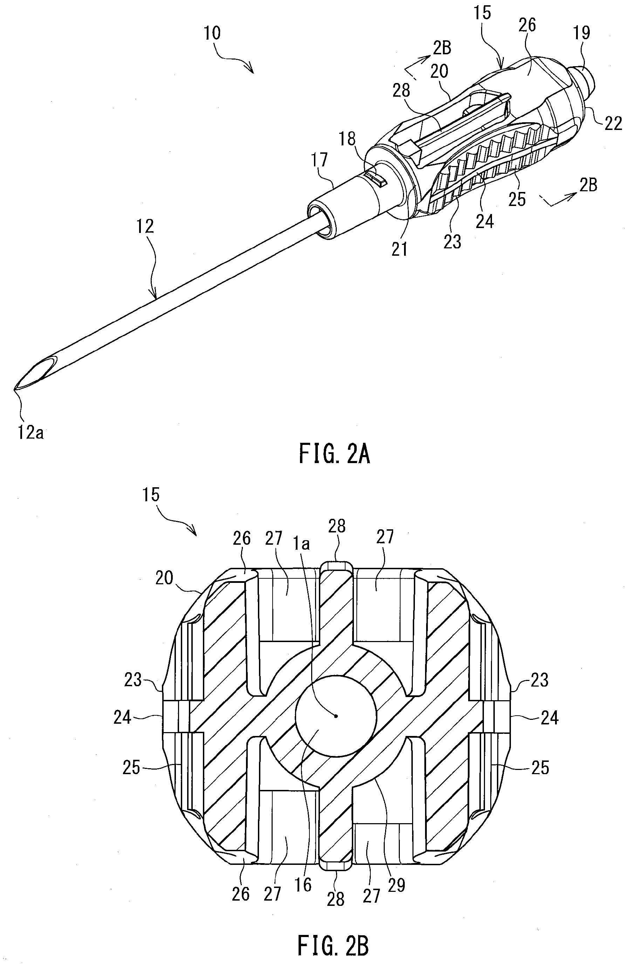

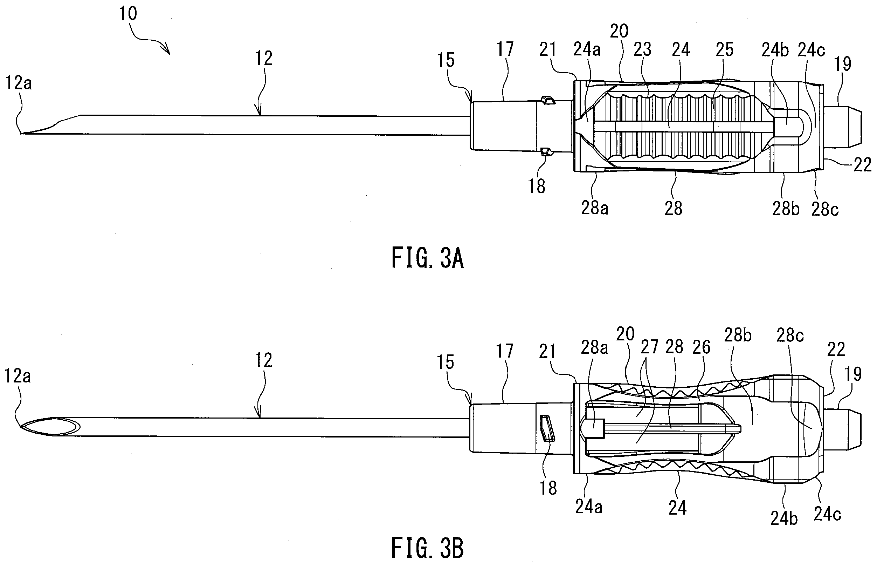

FIG. 2A is a perspective view of the needle unit 10. FIG. 2B is a cross-sectional view of the needle unit 10 taken along a plane containing line 2B-2B in FIG. 2A. FIG. 3A is a side view of the needle unit 10, and FIG. 3B is a top view of the needle unit 10. The needle unit 10 includes an injection needle 12 and a hub 15 that holds a base end of the injection needle 12. FIG. 2B is a cross-sectional view of the hub 15. In FIG. 2B, reference numeral 1a denotes a central axis of the needle unit 10. The central axis 1a extends along and coaxially with the injection needle 12. The central axis 1a also serves as the central axis of the needle device 1. For the sake of convenience of description below, the direction of a straight line that is perpendicular to the central axis 1a will be referred to as the "radius direction" or "radial direction". In the radius direction, a side that is closer to the central axis 1a is referred to as the "inner side", and a side that is away from the central axis 1a is referred to as the "outer side". A direction that is parallel to the central axis 1a is referred to as the "front-rear direction". The "front" side refers to a side that is to be inserted into a patient, and the "rear" side refers to an opposite side to the front side. These terms apply to the needle device 1 and various members constituting the needle device 1.

The injection needle 12 is made of a hard material such as a metal. The injection needle 12 has a hollow, substantially circular cylindrical shape, and a tip 12a thereof is sharpened.

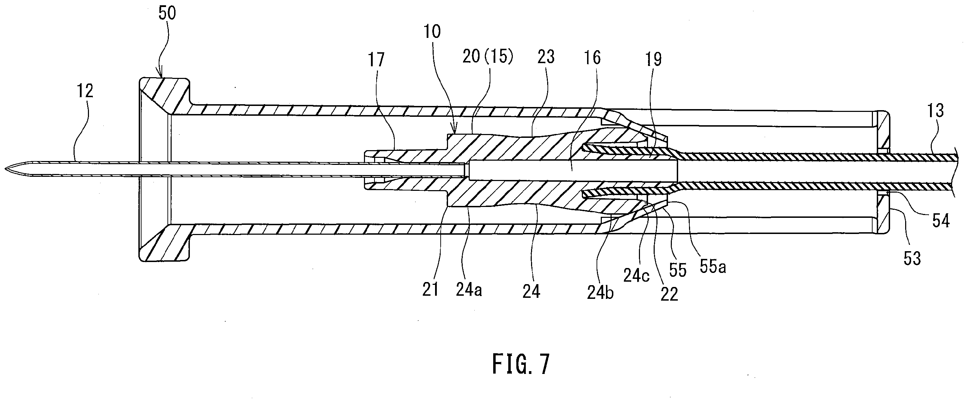

The hub 15 includes a small diameter portion 17, a cylindrical portion 19, and a hub main body 20 therebetween. A through hole 16 that is coaxial with the central axis 1a penetrates the hub 15 along a longitudinal direction thereof (see FIGS. 7 to 9, which will be describe later). The small diameter portion 17 and the cylindrical portion 19 are in communication with each other via the through hole 16.

Abase end (or rear end, that is, an end opposite to the tip 12a of the injection needle 12) of the injection needle 12 is inserted in the small diameter portion 17. The injection needle 12 is in communication with the through hole 16 of the hub 15. The small diameter portion 17 has a hollow, substantially circular cylindrical shape that is coaxial with the central axis 1a. A pair of helical protrusions 18 are provided on an outer circumferential surface of the small diameter portion 17. The helical protrusions 18 extend helically around the central axis 1a and function as external threads.

The hub main body 20 has a larger outer diameter than the small diameter portion 17 and the cylindrical portion 19. An outer circumferential end edge of the hub main body 20 on the front side (small diameter portion 17 side) has a circular shape that is concentric with the central axis 1a. In the present invention, this outer circumferential end edge is referred to as a shoulder portion 21. The outer diameter of the shoulder portion 21 is larger than the outer diameter of the small diameter portion 17 that is located forward of and adjoins the shoulder portion 21. In other words, the shoulder portion 21 protrudes further outward in the radius direction than the small diameter portion 17. An outer circumferential rear end edge 22 of the hub main body 20 on the rear side (cylindrical portion 19 side) also has a circular shape that is concentric with the central axis 1a. The outer diameter of the outer circumferential rear end edge 22 is larger than the outer diameter of the cylindrical portion 19.

A portion of the hub main body 20 between the shoulder portion 21 and the outer circumferential rear end edge 22 has a substantially quadrangular prism shape. Four lateral faces constituting the substantially quadrangular prism shape include a pair of opposing first lateral faces 23 and a pair of opposing second lateral faces 26.

The first lateral faces 23 are provided to allow a medical personnel to grip the needle unit 10 with two fingers when inserting the injection needle 12 into a patient. In order that the medical personnel can firmly grip the first lateral faces 23, the first lateral faces 23 are each provided with an inward curved surface whose middle portion in the front-rear direction (direction of the central axis 1a) is relatively recessed (i.e., depressed) inward in the radius direction compared with two end portions in the front-rear direction. Moreover, in order to prevent the fingers from slipping, the inward curved surface is provided with a protruding and recessed shape 25 in which rib-like protrusions and grooves that extend in a direction perpendicular to the front-rear direction are alternately arranged in the front-rear direction. Furthermore, the inward curved surface is provided with a rib 24 that extends in the front-rear direction. The rib 24 intersects the rib-like protrusions and grooves constituting the protruding and recessed shape 25. The rib 24 protrudes further outward in the radius direction than the rib-like protrusions constituting the protruding and recessed shape 25. A top surface (surface facing outward in the radius direction) of the rib 24 is a smooth, inward curved surface whose middle portion in the front-rear direction (direction of the central axis 1a) is depressed as is the case with the above-described inward curved surface. Aflat surface 24a smoothly connects a front end of the rib 24 to the shoulder portion 21. The flat surface 24a is a plane that is substantially parallel to the central axis 1a. Aflat surface 24b and a sloping surface 24c smoothly connect a rear end of the rib 24 to the outer circumferential rear end edge 22. The flat surface 24b is a plane that is substantially parallel to the central axis 1a. The sloping surface 24c is an outward curved surface that slopes from the outer circumferential rear end edge 22 toward the flat surface 24b in a direction away from the central axis 1a, and is of the same kind as a so-called "chamfer".

When inserting the injection needle 12 into a blood vessel of a patient, with, for example, the thumb and the forefinger gripping the pair of first lateral faces 23, one of the pair of second lateral faces 26 opposes the skin of the patient, and the other second lateral face 26 faces the opposite side to the patient. When the tip 12a of the injection needle 12 is inserted into the blood vessel, the blood flows into the through hole 16 of the hub 15 through the injection needle 12. This phenomenon is called "flashback". In order to make it easy for the medical personnel to visually confirm a flashback, as shown in FIG. 2B, each second lateral face 26 is provided with a groove 27 that reaches a conduit 29 that defines the through hole 16. A rib 28 extending in the front-rear direction is provided within the groove 27 so as to divide the groove 27 into two sections. In a region where the groove 27 is present with respect to the front-rear direction, the rib 28 protrudes outward in the radius direction the furthest in the second lateral face 26. A top surface (surface facing outward in the radius direction) of the rib 28 is a flat surface that is substantially parallel to the central axis 1a. Aflat surface 28a smoothly connects a front end of the rib 28 to the shoulder portion 21. The flat surface 28a is a plane that is substantially parallel to the central axis 1a. A flat surface 28b and a sloping surface 28c smoothly connect a rear end of the rib 28 to the outer circumferential rear end edge 22. The flat surface 28b is a plane that is substantially parallel to the central axis 1a. The sloping surface 28c is a plane or an outward curved surface that slopes from the outer circumferential rear end edge 22 toward the flat surface 28b in a direction away from the central axis 1a, and is of the same kind as a so-called "chamfer".

The sloping surfaces 24c each extend in a circumferential direction so as to connect the two sloping surfaces 28c of the second lateral faces 26 to each other. The sloping surfaces 24c may have, for example, the shape of a portion of a conical surface or the shape of a portion of a spherical surface, or a shape that is close to these shapes. The sloping surfaces 24c and 28c smoothly connect the lateral faces 23 and 26 that constitute the substantially quadrangular prism shape to the circular outer circumferential rear end edge 22. In the present embodiment, the sloping surfaces 24c and the sloping surfaces 28c are adjacent to each other via clear boundaries. However, the sloping surfaces 24c and the sloping surfaces 28c may be continuous with each other via curved surfaces that are so smooth that the boundaries are unclear.

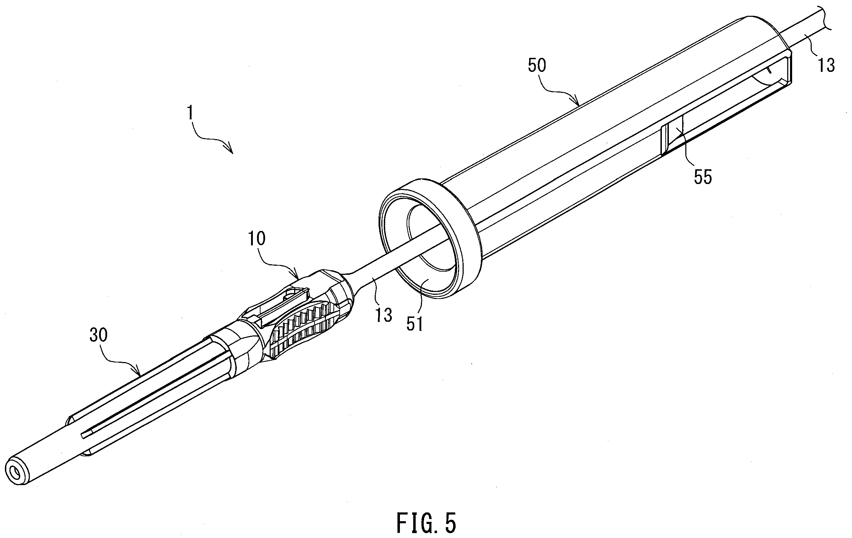

The cylindrical portion 19 is connected to a flexible tube 13 (see FIGS. 5 to 9, which will be described later).

There is no limitation on the material of the hub 15, and it is preferable to use a hard material. For example, resin materials such as polycarbonate, polypropylene, and polyethylene can be used.

Referring back to FIG. 1, the cover 30 has a hollow, substantially circular cylindrical shape as a whole. One end (rear end) 31 of the cover 30 in the longitudinal direction is open, while the other end (front end) is closed. An outer circumferential end edge of the rear end 31 of the cover 30 has a circular shape. An outer circumferential surface 32 near the rear end 31 is a substantially circular cylindrical surface. The outer diameter of the rear end 31 of the cover 30 is slightly larger than the outer diameter of the shoulder portion 21 of the hub 15. An internal thread 38 is provided on an inner circumferential surface of the cover 30 near the rear end 31. The internal thread 38 is configured to be able to be screwed to the helical protrusions 18 provided on the small diameter portion 17 of the hub 15.

There is no limitation on the material of the cover 30, and it is preferable to use a hard material. For example, resin materials such as polycarbonate, polypropylene, and polyethylene can be used.

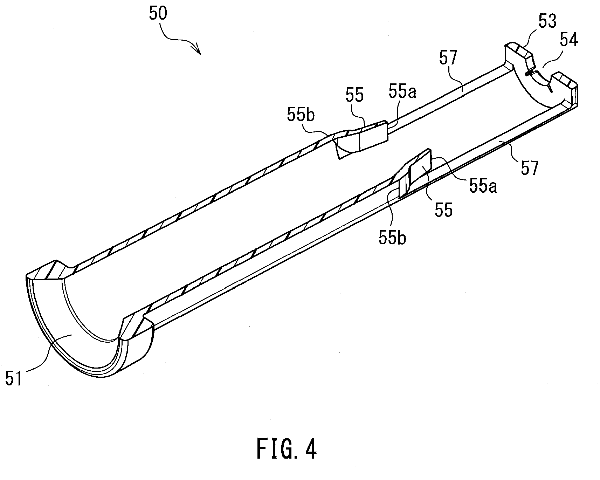

FIG. 4 is a cross-sectional perspective view of the protector 50. The protector 50 has a hollow, substantially circular cylindrical shape as a whole. An inner circumferential surface of the protector 50 is a substantially circular cylindrical surface. The protector 50 has on its front side an opening 51 through which the needle unit 10 can be inserted. A stopper plate 53 is provided at a rear end of the protector 50. A hole 54 having a smaller diameter than the opening 51 is provided in the center of the stopper plate 53.

A pair of locking claws 55 are provided on side walls of the protector 50 so as to oppose each other. Each locking claw 55 is an elongated plate-shaped member and has a cantilevered structure including a free end 55a, which is a leading end of the locking claw 55, and a fixed end 55b that is arranged forward of (closer to the opening 51 than) the free end 55a. Each locking claw 55 slopes from the fixed end 55b toward the free end 55a in an inward radius direction. The leading end (free end) 55a of the locking claw 55 protrudes inward from the inner circumferential surface of the protector 50. The locking claw 55 can be elastically deformed to bend so that the leading end 55a can be displaced outward in the radius direction.

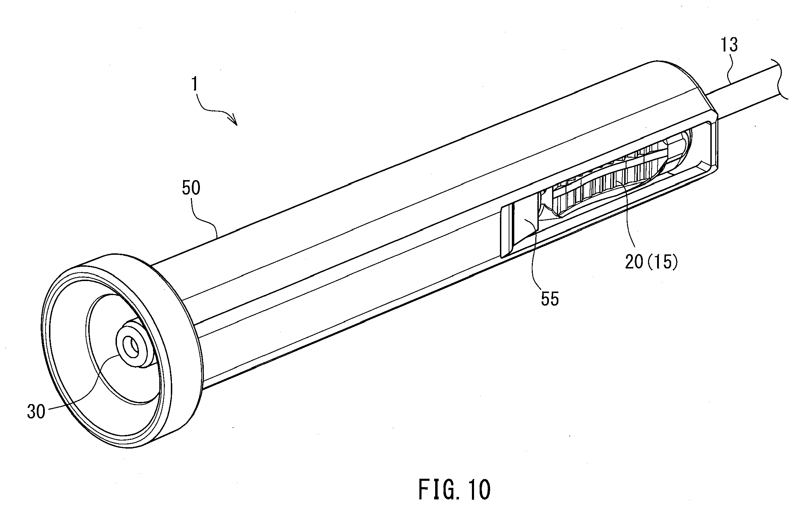

A window 57 via which an inner cavity of the protector 50 is in communication with the outside is provided in each of the side walls of the protector 50 and located between the locking claw 55 and the stopper plate 53. The needle unit 10 (in particular, its hub 15) when contained in the protector 50 can be directly visually observed through the window 57 (see FIG. 10, which will be described later).

The inner cavity of the protector 50 is in communication with the outside only via the opening 51, the hole 54, and the windows 57. Note that, in the present invention, the windows 57 are not necessarily needed, and may be omitted.

There is no limitation on the material of the protector 50, and it is preferable to use a hard material. Moreover, it is preferable that the material of the protector 50 is flexible so that the locking claws 55 can be deformed. For example, resin materials such as polypropylene and polyethylene can be used.

A method of using the needle device 1 of the present embodiment that is configured as described above will be described.

FIG. 5 is a perspective view of the needle device 1 before use. The cover 30 is attached to the needle unit 10. The injection needle 12 is contained in an inner cavity of the cover 30. The helical protrusions 18 (see FIG. 2A) of the hub 15 are screwed to the internal thread 38 (see FIG. 1) formed on the inner circumferential surface of the cover 30 near the rear end 31. The flexible tube 13 is connected to the cylindrical portion 19 of the hub 15. The injection needle 12 is in communication with the tube 13 via the through hole 16 of the hub 15 (see FIGS. 7 to 9, which will be described later). The tube 13 is inserted into the protector 50 through the opening 51 and is led from the hole 54 (see FIG. 4) provided in the stopper plate 53. The protector 50 can be freely moved on the tube 13 toward the needle unit 10, or away from the needle unit 10.

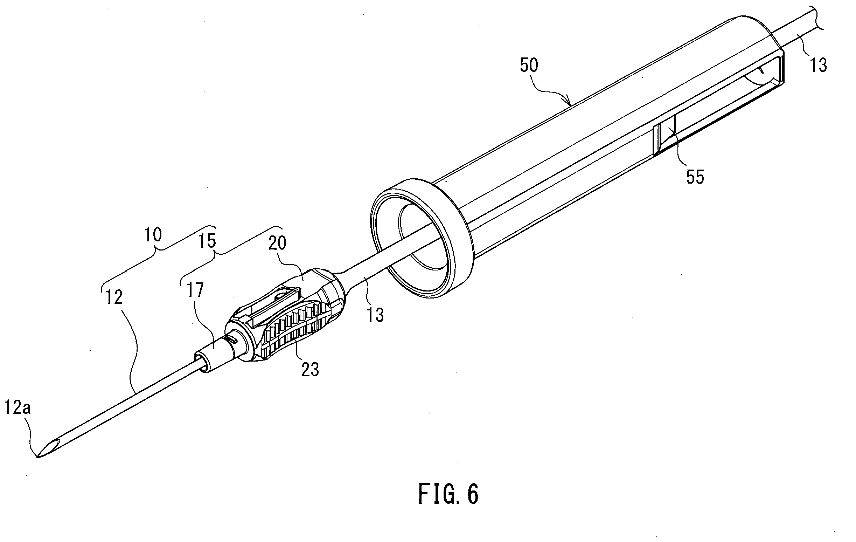

Next, as shown in FIG. 6, the cover 30 is detached from the needle unit 10. The first lateral faces 23 of the hub 15 are gripped, and the tip 12a of the injection needle 12 is inserted into a blood vessel of a patient. A required procedure, such as blood collection, blood transfusion, or infusion, is performed. After that, the injection needle 12 is withdrawn from the patient.

Next, the protector 50 is gripped with one hand, the tube 13 led from the hole 54 of the protector 50 is gripped with the other hand, and the tube 13 is pulled. The needle unit 10 is retracted into the protector 50 from the hub 15 side. The sloping surfaces 24c and 28c (see FIGS. 3A and 3B) near the rear end of the hub 15 reduce the likelihood of the bub 15 colliding with or getting caught on an end edge of the protector 50 that defines the opening 51. Therefore, the hub 15 can be easily retracted into and contained in the protector 50 simply by pulling the tube 13 without the need to touch the protector 50 with the hand.

FIG. 7 is a cross-sectional view showing a state in which the needle unit 10 is in the process of being retracted into the protector 50. The needle unit 10 is moving within the protector 50 toward the stopper plate 53. The direction in which the pair of first lateral faces 23 oppose each other and the direction in which the pair of locking claws 55 oppose each other are the same.

As described above, the locking claws 55 slope such that the leading ends 55a of the locking claws 55 protrude inward from the inner circumferential surface of the protector 50. The distance between the leading ends 55a of the opposing locking claws 55 is smaller than the outer size of the hub main body 20. Accordingly, when the hub main body 20 passes between the leading ends 55a of the locking claws 55, the hub main body 20 hits the locking claws 55 and elastically deforms the locking claws 55 to bend such that the leading ends 55a move outward in the radius direction. The leading ends 55a of the locking claws 55 slide on the hub main body 20 in the order of the sloping surfaces 24c, the flat surfaces 24b, the ribs 24, the flat surfaces 24a, and the shoulder portion 21 (see FIGS. 3A and 3B). Since these portions are smoothly continuous with each other, the leading ends 55a do not get caught on the hub main body 20 in the process of sliding. Since the sloping surfaces 24c are provided near the rear end of the hub main body 20, a relatively small pulling force applied to the tube 13 will suffice to initially deform the locking claws 55 to bend largely.

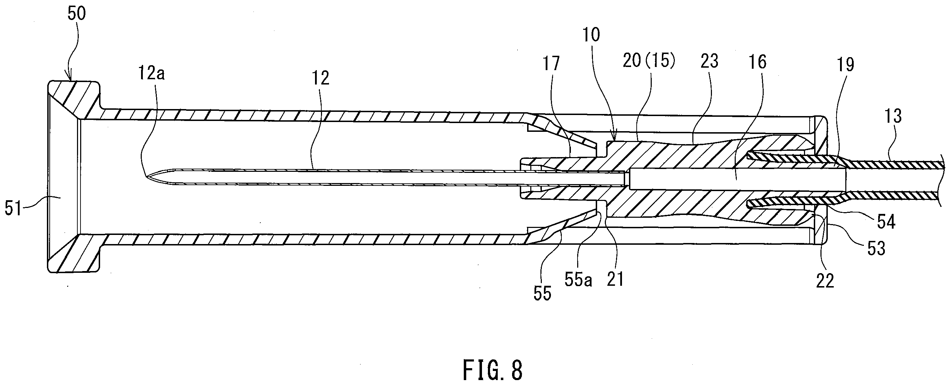

When the leading ends 55a of the locking claws 55 finish passing the shoulder portion 21, the locking claws 55 elastically return as shown in FIG. 8. The distance between the leading ends 55a of the opposing locking claws 55 is smaller than the outer diameter of the shoulder portion 21 of the hub 15. Therefore, the locking claws 55 engage with the hub 15 (in particular, its shoulder portion 21) in the front-rear direction. The outer circumferential rear end edge 22 of the hub 15 abuts against or is located near the stopper plate 53 in the front-rear direction. Accordingly, the movement of the needle unit 10 in the front-rear direction relative to the protector 50 is restricted. The needle unit 10 cannot be separated from the protector 50.

The used needle device 1 is disposed of in the state shown in FIG. 8. The tip 12a of the injection needle 12 is located at a position rearward of the end edge of the opening 51 of the protector 50. Therefore, the likelihood of the occurrence of accidental puncture of the medical personnel's finger or the like by the tip 12a of the used injection needle 12 (accidental needle stick) is low.

As described above, the needle device disclosed in Patent Document 1 has the problem in that, if an unused needle unit is accidentally contained in the protector, the needle unit cannot be pulled out of the protector thereafter. The needle device 1 of the present embodiment solves this problem. This will be described below.

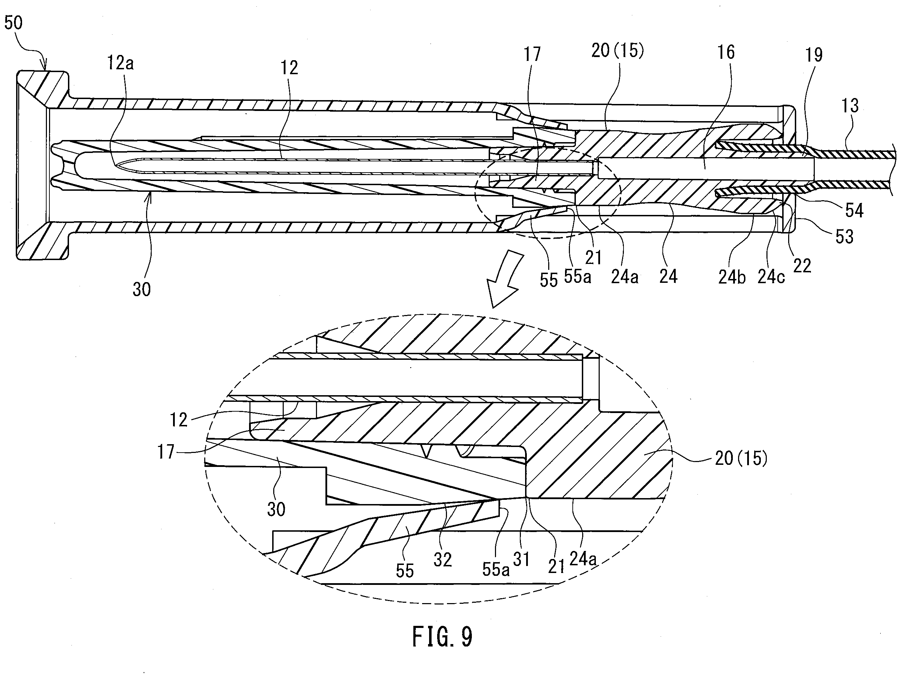

In the needle device 1 before use shown in FIG. 5, the needle unit 10 can be contained in the protector 50. FIG. 9 is a cross-sectional view showing a state in which the unused needle unit 10 is contained in the protector 50. Unlike FIG. 8, FIG. 9 shows the needle unit 10 to which the cover 30 is attached. The rear end 31 of the cover 30 and the shoulder portion 21 of the hub 15 adjoin each other in the front-rear direction. When the needle unit 10 is inserted furthest into (i.e., to the rearmost portion of) the protector 50, the leading ends 55a of the locking claws 55 abut against the outer circumferential surface 32 near the rear end 31 of the cover 30. As described above, the outer diameter of the rear end 31 of the cover 30 is slightly larger than the outer diameter of the shoulder portion 21 of the hub 15. Therefore, the rear end 31 of the cover 30 protrudes slightly further outward in the radius direction than the shoulder portion 21 of the hub 15. Since the locking claws 55 slope radially inward toward the leading ends 55a, the leading ends 55a of the locking claws 55 can easily pass over the radial step between the shoulder portion 21 and the rear end 31 in the process through which the needle unit 10 is retracted into and contained in the protector 50.

In the state shown in FIG. 9, the tube 13 led from the hole 54 of the protector 50 is pushed into the protector 50. The needle unit 10 moves forward within the protector 50. Since the shoulder portion 21 of the hub 15 is located inward of the rear end 31 of the cover 30 in the radius direction, the leading ends 55a of the locking claws 55 can easily move from the rear end 31 of the cover 30 to the shoulder portion 21 of the hub 15. After that, the leading ends 55a of the locking claws 55 slide on the hub 15 in the order of the flat surfaces 24a, the ribs 24, the flat surfaces 24b, and the sloping surfaces 24c (see FIGS. 3A and 3B). The leading ends 55a of the locking claws 55 do not get caught on the hub 15 in the process of sliding. Accordingly, the needle unit 10 can be pushed out from the opening 51 of the protector 50, so that the needle unit 10 can be separated from the protector 50 as shown in FIG. 5.

As described above, with the needle device 1 of the present embodiment, in a state in which the cover 30 is attached to the needle unit 10, the needle unit 10 can be repeatedly inserted into and removed from the protector 50 any number of times. Therefore, even if an unused needle unit 10 is contained in the protector 50, the needle device 1 does not become unusable. On the contrary, an unused needle device 1 can be stored in a state in which the needle unit 10 to which the cover 30 is attached is contained in the protector 50 as shown in FIG. 10. This makes the entire needle device 1 compact, and is therefore advantageous in reducing the storage space for the needle device 1 and improving the handleability thereof. Moreover, since the cover 30 is contained in the protector 50, the likelihood of unintentional separation of the cover 30 from the needle unit 10 caused by, for example, a worker touching the cover 30 when handling the needle device 1 is reduced.

In the above-described example, the needle unit 10 is inserted into the protector 50 such that the locking claws 55 slide on the first lateral faces 23 of the hub 15. However, the needle device 1 of the present embodiment is configured such that the needle unit 10 can also be inserted into the protector 50 such that the locking claws 55 slide on the second lateral faces 26. In this case as well, similar effects to those of the above-described example can be achieved. The restrictions on the position of the needle unit 10 in a rotational direction around the central axis 1a during the insertion into the protector 50 are eased, and therefore, the operation for retracting the used needle unit 10 into the protector 50 can be performed in a straightforward and quick manner.

The first lateral faces 23 are provided with the ribs 24, and the second lateral faces 26 are provided with the ribs 28. When the needle unit 10 moves within the protector 50 in the front-rear direction, the locking claws 55 slide on the ribs 24 or the ribs 28. Therefore, a situation in which the locking claws 55 get caught on the protruding and recessed shapes 25 of the first lateral faces 23 or end edges of the grooves 27 in the second lateral faces 26 and obstruct the movement of the needle unit 10 relative to the protector 50 does not arise.

The above-described embodiment is given for illustrative purpose only. The present invention is not limited to the above-described embodiment, and changes can be made thereto as appropriate.

In the above-described embodiment, the outer diameter of the rear end 31 of the cover 30 is slightly larger than the outer diameter of the shoulder portion 21 of the hub 15. However, these outer diameters may be equal to each other. In this case, when the cover 30 is attached to the needle unit 10, the rear end 31 of the cover 30 and the shoulder portion 21 of the hub 15 constitute a smooth, continuous surface, so that the needle unit 10 to which the cover 30 is attached can be freely inserted into and removed from the protector 50 as is the case with the above-described embodiment. Note that, normally, a configuration in which the rear end 31 of the cover 30 protrudes further outward in the radius direction than the shoulder portion 21 of the hub 15 as in the above-described embodiment is advantageous in reducing the likelihood of the leading ends 55a of the locking claws 55 getting caught on the shoulder portion 21 when the needle unit 10 with the cover 30 is pushed out of the protector 50.

The configurations of the first lateral faces 23 and the second lateral faces 26 of the hub 15 are not limited to those of the above-described embodiment. For example, one or both of the ribs 24 and the ribs 28 may extend from the shoulder portion 21 to the outer circumferential rear end edge 22. The first lateral faces 23 may not be provided with one or both of the inward curved surfaces and the protruding and recessed shapes 25. In the case where the first lateral faces 23 are constituted by smooth, flat or curved surfaces without the protruding and recessed shapes 25, the ribs 24 may be omitted. Moreover, the second lateral faces 26 may not be provided with the grooves 27. In the case where the second lateral faces 26 are constituted by smooth, flat or curved surfaces, the ribs 28 may be omitted.

In the above-described embodiment, the cover 30 is configured to be able to be securely fixed to the hub 15 by screwing the internal thread 38 to the helical protrusions 18. However, the present invention is not limited to this configuration, and the internal thread 38 and the helical protrusions 18 may be omitted. For example, a configuration may also be employed in which a female tapered surface is formed on the inner circumferential surface of the cover 30, a male tapered surface is formed on the small diameter portion 17 of the hub 15, and the cover 30 is attached to the hub 15 by fitting the female tapered surface and the male tapered surface to each other (so-called taper fitting).

### Industrial Applicability

There is no particular limitation on the field of application of the present invention, and the present invention can be widely used as needle devices that are inserted into blood vessels during procedures such as blood collection, blood transfusion, infusion, and extracorporeal blood circulation.

### List of Reference Numerals

- 1: Needle device
- 10: Needle unit
- 12: Injection needle
- 12a: Tip of injection needle
- 13: Tube
- 15: Hub
- 20: Hub main body
- 21: Shoulder portion
- 24, 28: Rib
- 24c, 28c: Sloping surface
- 25: Protruding and recessed shape
- 30: Cover
- 31: Rear end of cover
- 50: Protector
- 55: Locking claw
- 55a: Free end (leading end of locking claw)
- 55b: Fixed end

## Claims

1. A needle device comprising:
a needle unit having an injection needle with a sharp tip, and a hub holding a base end of the injection needle;
a cover detachably attached to the needle unit so as to contain the injection needle; and
a hollow, cylindrical protector having a locking claw,
the needle device being configured such that the needle unit can be retracted into and contained in the protector from the hub side,
wherein, in a state in which the cover is not attached to the needle unit, once the needle unit is contained in the protector, the locking claw engages with the hub so that the needle unit cannot be separated from the protector, and
in a state in which the cover is attached to the needle unit, the needle unit can be inserted into and removed from the protector.

2. The needle device according to claim 1,
wherein the locking claw has a cantilevered structure including a free end that is a leading end of the locking claw and a fixed end that is arranged forward of the free end, and the locking claw slopes from the fixed end toward the free end in an inward radius direction.

3. The needle device according to claim 1 or 2,
wherein the locking claw is elastically deformable such that the leading end thereof is displaced outward in a radius direction.

4. The needle device according to any one of claims 1 to 3,
wherein the hub has a shoulder portion that adjoins a rear end of the cover when the cover is attached to the needle unit,
the shoulder portion is configured such that, in a state in which the cover is not attached to the needle unit, the locking claw engages with the shoulder portion, and
in a state in which the cover is attached to the needle unit, the rear end of the cover protrudes further outward in a radius direction than the shoulder portion.

5. The needle device according to any one of claims 1 to 4,
wherein an outer circumferential surface of the hub is provided with a rib extending in a direction in which the needle unit moves relative to the protector, and
the rib is configured such that, when the needle unit moves within the protector, the locking claw slides on the rib.

6. The needle device according to claim 5,
wherein the outer circumferential surface of the hub provided with the rib is provided with a protruding and recessed shape for preventing slipping when gripped, and
the rib protrudes further outward in a radius direction than the protruding and recessed shape.

7. The needle device according to any one of claims 1 to 6,
wherein a sloping surface is provided near a rear end of an outer circumferential surface of the hub, the sloping surface sloping forward in a direction away from a central axis, and
the sloping surface is configured such that, when the needle unit moves within the protector, the locking claw slides on the sloping surface.

8. The needle device according to any one of claims 1 to 7, further comprising
a flexible tube connected to a rear end of the hub so as to be in communication with the injection needle, and
the tube penetrates the protector.
